# EUROPÄISCHE PATENTSCHRIFT

(11) **EP 3 668 377 B1**
(45) Veröffentlichungstag und Bekanntmachung des Hinweises auf die Patenterteilung: **01.06.2022**
(21) Anmeldenummer: 18762031.5
(22) Anmeldetag: 16.08.2018
(51) Int. Cl.: A61B 5/00, A61B 5/05

(54) **VORRICHTUNG ZUR ZUSTANDSERFASSUNG AN PASSIVIERUNGSSCHICHTEN EINER VERKAPSELUNG**
DEVICE FOR DETERMINING THE STATUS OF PASSIVATION LAYERS OF AN ENCAPSULATION
DISPOSITIF DE DÉTERMINATION D'UN ÉTAT AU NIVEAU DE COUCHES DE PASSIVATION D'UNE ENCAPSULATION

(30) Priorität: 16.08.2017 DE 102017118686
(43) Veröffentlichungstag der Anmeldung: 24.06.2020
(73) Patentinhaber: Aesculap AG, 78532 Tuttlingen (DE); Eberhard Karls Universität Tübingen, 72074 Tübingen (DE)
(72) Erfinder: HOFMANN, Boris, 78582 Balgheim (DE); WESTERHAUSEN, Markus, 72762 Reutlingen (DE)
(74) Vertreter: Winter, Brandl - Partnerschaft mbB
(86) Internationale Anmeldenummer: PCT/EP2018/072265
(87) Internationale Veröffentlichungsnummer: WO 2019/034749

(56) Entgegenhaltungen:
- US-A- 5 750 926
- US-A1- 2002 042 561
- US-A1- 2003 181 953
- US-A1- 2005 159 800
- US-A1- 2009 308 762

## Beschreibung

Die Erfindung betrifft ein Fehleranalyse-System nach Anspruch 1 zur Erfassung eines Zustands von Verkapselungen und/oder Passivierungsschichten, und bezieht sich insbesondere auf ein System nach Anspruch 1 zur elektrischen Messung an leitenden, zwischen Passivierungsschichten einer Verkapselung angeordneten Zwischenschichten und deren Auswertung in Bezug auf einen funktionellen Zustand einer Passivierungsschicht.

Für Therapie- und Diagnoseverfahren sind beispielsweise elektrisch aktive und/oder flexible Implantate bekannt. Die Verkapselung derartiger flexibler Implantate muss biostabil, biokompatibel sowie elektrisch isolierend sein.

Gängige Polymere, wie Polyimid oder Parylen (Poly-p-Xylylen), stellen zumindest diese Eigenschaften sicher, sind allerdings in kleinen Mengen wasserdampfdurchlässig und mithin nicht vollständig diffusionsdicht. Über längere Zeit hinweg führt dies zu etlichen Degradationseffekten am Implantat.

Durch Einbetten wasserdampfundurchlässiger Schichten bzw. Zwischenschichten in Passivierungsschichten oder Verringern einer Delamination isolierender Schichten von zwischenliegenden wasserdampfundurchlässigen Schichten bzw. Layern durch spezielle Adhäsionslayer, die eine Adhäsion an andere Werkstoffe erhöhen, kann zwar die Langzeitstabilität der flexiblen Verkapselung und damit des flexiblen Implantates verbessert werden. Das Dokument US 2009/0308762 A1 offenbart elektrische Messverfahren zur Bestimmung der Dichtheit von verkapselten Implantaten.

Bislang ist es jedoch nicht möglich, eine in vivo Überwachung der Passivierung durchzuführen und (Echtzeit)-Aussage über den Zustand der flexiblen Verkapselungen zu treffen. Verkapselungen bzw. Implantate können zurzeit lediglich als entweder noch funktionsfähig und damit isolierend, oder defekt und somit schlimmstenfalls von elektrisch aktiven Schichten getrennt, erfasst werden. Eine fortschreitende, bislang nicht beobachtbare Alterung der Passivierung führt ohne erfassbare Erkennbarkeit durch beispielsweise auslesbare Signalisierung in der Regel zu einem plötzlichen, und damit unerwünschten, Ausfall des Implantats.

Vor diesem Hintergrund liegt der Erfindung als eine Aufgabe zugrunde, ein Verfahren zur Zustandserfassung an Passivierungsschichten einer Verkapselung bereitzustellen, mit welchem Degradationseffekte an flexiblen passivierenden Schichten frühzeitig erfassbar sind.

Diese Aufgabe wird erfindungsgemäß durch ein Verfahren zur Erfassung und/oder Vorhersage eines Zustands von Verkapselungsschichten und/oder Passivierungsschichten mit den Merkmalen des Anspruchs 1 gelöst. Vorteilhafte Weiterbildungen der Erfindung sind Gegenstand der beigefügten Unteransprüche.

Der Erfindung liegt die allgemeine Idee zugrunde, durch eine elektrische Analyse und/oder elektrische Messungen und anschließende Bewertung (einzelner) passivierender Schichten mittels eingebrachter Zwischenlagen negative Degradationseffekte innerhalb dieser Passivierung frühzeitig zu erkennen. Beispielsweise kann eine elektrische Ankontaktierung von Zwischenlagen aus einem leitfähigen Material in einem passivierenden Mehrschichtsystem an einem zu schützenden Gegenstand, beispielsweise einem flexiblen Implantat, oder an flexiblen oder nichtflexiblen Polyimidschichten mit zwischenliegenden Strukturen aus einem leitfähigen Material die Analyse zumindest einer passivierenden Schicht erlauben. Dies kann bereits im Vorfeld zur Bestimmung/Überprüfung eines Produktionsausschusses geschehen, erlaubt jedoch auch eine Echtzeitüberwachung im Hinblick auf Materialermüdung, insbesondere während das Implantat in vivo bereits im Körper verweilt. Dadurch kann vorteilhaft eine Verschlechterung der passivierenden Eigenschaften einzelner Layer im Voraus detektiert werden, während das gesamte System als solches noch stabil ist, und werden eine in vivo Fehler-/Gesundheitsanalyse einer (noch funktionierenden) Implantatpassivierung sowie eine Ermittlung eines Produktionsausschusses ohne Zuhilfenahme externer Referenzen möglich.

In anderen Worten wird erfindungsgemäß vorgeschlagen, zu einem vorbestimmten Anfangszeitpunkt und sodann in einem Verwendungszustand fortlaufend die einzelnen Passivierungsschichten über eine Strommessung auf Beeinträchtigung, d.h. Verschlechterung ihrer Eigenschaften, zu überwachen. Eine selektive Beaufschlagung der Ankontaktierungen der Zwischenschichten und/oder zumindest einer Elektrode bzw. einem Erfassungspunkt des zu schützenden Objekts, vorzugsweise Implantat, mit einem elektrischen Signal, beispielsweise einer Spannung eines vorbestimmten Werts (beispielsweise 1V), gegen eine Referenzkomponente bzw. ein Referenzpotenzial (systemextern im Anfangszeitpunkt oder systemintern in vivo bzw. bei Verwendung) erlaubt eine selektive Messung an jeder Passivierungsschicht und damit eine Aussage darüber, ob eine Beeinträchtigung einer Passivierungsschicht vorliegt und falls ja, welche der Passivierungsschichten beeinträchtigt ist. Da ein Zeitraum, über welchen hinweg beispielsweise eine oberste Passivierungsschicht ihre passivierende Eigenschaft verliert, bekannt oder zumindest empirisch näherungsweise bestimmbar ist (beispielsweise 60,5 Tage im hier zugrunde liegenden Beispiel), kann ein Zeitpunkt systemrelevanter Ausfall einzelner Passivierungsschichten oder auch des Gesamtsystems bzw. Implantats zumindest näherungsweise vorhergesagt oder abgeschätzt werden. Darüber hinaus ermöglicht die anfängliche Messung am Ende des Herstellungsprozesses, diejenigen Systeme bereits vor ihrer Verwendung auszusortieren, für welche ein vorzeitiger Ausfall als wahrscheinlich erkennbar ist.

Im Einzelnen beinhaltet ein nicht erfindungsgemäßes und nur der Veranschaulichung dienendes Verfahren zur Erfassung eines Zustands einer Verkapselung und/oder einer Passivierungsschicht der Verkapselung, wobei die Verkapselung ein Mehrschichtsystem aus mehreren wechselweise übereinander liegend angeordneten Passivierungsschichten und zwischen den Passivierungsschichten liegend angeordneten, elektrisch ankontaktierten Zwischenschichten bildet, das Mehrschichtsystem dazu angeordnet ist, ein von der Verkapselung umgebenes Objekt, vorzugsweise Implantat, zu schützen, und eine oberste Schicht und eine unterste Schicht des Mehrschichtsystems durch jeweils eine oberste und eine unterste Passivierungsschicht gebildet wird, die Schritte: Durchführen einer elektrischen Messung unter Verwendung eines elektrischen Signals zwischen einem Referenzpotenzial und zumindest einer elektrisch ankontaktierten Zwischenschicht, und Erfassen zumindest eines zwischen dem Referenzpotenzial und der zumindest einen elektrisch ankontaktierten Zwischenschicht fließenden Stroms; und Vergleichen des zumindest einen erfassten Stroms mit zumindest einem vorbestimmten Schwellenwert, wobei ein Unterschreiten oder ein Überschreiten des zumindest einen Schwellenwerts einen funktionellen Zustand einer zu der zumindest einen elektrisch ankontaktierten Zwischenschicht benachbarten Passivierungsschicht anzeigt. Unter einer elektrischen Messung sind hierin beispielsweise DC- bzw. Gleichsignalmessungen unter Verwendung eines elektrischen Gleichsignals oder AC- bzw. Wechselsignalmessungen unter Verwendung eines Wechselsignals zu verstehen.

Bevorzugt werden bei dem vorgenannten nicht erfindungsgemäßen Verfahren mehrere jeweils elektrisch ankontaktierte Zwischenschichten, die zwischen jeweils zwei Passivierungsschichten der Verkapselung angeordnet sind, gegen ein gemeinsames Referenzpotenzial gemessen; wird für jede der mehreren Zwischenschichten jeweils ein Messstrom erfasst, der einer entsprechenden der Passivierungsschichten zugeordnet ist und deren funktionellen Zustand anzeigt; und wird jeder Messstrom mit jeweils einem vorbestimmten Schwellenwert verglichen, wobei ein Überschreiten des vorbestimmten Schwellenwerts einen beeinträchtigten Zustand der jeweils zugeordneten Passivierungsschicht anzeigt. Hierbei kann das gemeinsame Referenzpotenzial auch eine der Zwischenschichten selbst sein bzw. von dieser gebildet werden. Ein gemeinsames Potenzial kann extern mit Hilfe der Referenzelektrode angelegt und sodann über die Zwischenschichten abgegriffen werden, oder intern mit Hilfe der Zwischenschichten selbst angelegt werden. So kann beispielsweise an einer oberen Zwischenschicht ein Potenzial anliegen und dieses über eine mittlere Zwischenschicht abgegriffen werden, oder aber kann beispielsweise an einer unteren Zwischenschicht ein Potenzial anliegen und dieses über die mittlere Zwischenschicht abgegriffen werden.

Bevorzugt ist der vorbestimmte Schwellenwert null und zeigt ein Vergleich erfasster Messströme über mehrere Schichtfolgen hinweg einen Gesamtzustand des die Verkapselung bildenden Mehrschichtsystems an, wobei dann, wenn der Vergleich anzeigt, dass zumindest zwischen einem Erfassungspunkt des zu schützenden Objekts, vorzugsweise Implantats, und einer von diesem durch die unterste Passivierungsschicht getrennte Zwischenschicht kein Messstrom erfassbar ist, unabhängig von einem erfassten Zustand höher liegender Passivierungsschichten, auf funktionelle Stabilität des Gesamtsystems geschlossen wird.

Weiter bevorzugt ist der vorbestimmte Schwellenwert null und zeigt ein Vergleich erfasster Messströme über mehrere Schichtfolgen hinweg einen Gesamtzustand des die Verkapselung bildenden Mehrschichtsystems an, wobei dann, wenn das Mehrschichtsystem zumindest eine obere, eine mittlere und eine untere Passivierungsschicht beinhaltet, und wenn in einer anfänglichen Systemprüfmessung nach Produktionsende des Gesamtsystems ein messbarer Strom zwischen einer ersten Zwischenschicht, die zwischen der oberen und der mittleren Passivierungsschicht angeordnet ist, und einer zweiten Zwischenschicht, die zwischen der mittleren und der unteren Passivierungsschicht angeordnet ist, über die mittlere Passivierungsschicht erfasst wird, auf eine Ausschuss bedingende Grundbeeinträchtigung zumindest der mittleren Passivierungsschicht und/oder des Gesamtsystem geschlossen wird.

Bevorzugt ist bei dem vorstehenden nicht erfindungsgemäßen Verfahren die Verkapselung dazu angeordnet, ein Implantat, vorzugsweise ein flexibles Implantat, als das zu schützende Objekt, vorzugsweise Implantat, in vivo fluiddicht und/oder gasdicht zu kapseln, und ist das Verfahren dazu konfiguriert, anhand einer Analyse und Bewertung zumindest einer passivierenden Schicht durch elektrische Messung eine in vivo Fehleranalyse und/oder Gesundheitsanalyse der Implantatpassivierung und/oder Ermittlung von Produktionsausschuss unter Entfall externer Referenzkomponenten durchzuführen.

Bevorzugt wird die elektrische Messung externreferenzfrei zwischen zwei elektrisch ankontaktierten Zwischenschichten durchgeführt. Bei der elektrischen Messung vorzugsweise über die Zwischenschichten selbst wird an eine erste Zwischenschicht ein Potenzial angelegt und erfolgt ein Abgriff an einer zweiten Zwischenschicht. In einer darauffolgenden Messung, d.h. einer neuen Messung mit einem neuen Potenzial, wird das neue Potenzial sodann an beispielsweise die zweite Zwischenschicht angelegt und erfolgt ein Abgriff an einer dritten Zwischenschicht. Mittels elektrischen Messungen, die entsprechend der Anzahl vorhandener Zwischenschichten einem derartigen Ablauf folgen, wird vorteilhaft ein Produktionsausschuss erkennbar und eine qualitative Einstufung bzw. Beurteilung der einzelnen Passivierungsschichten möglich.

Bevorzugt wird die elektrische Messung an einer biostabilen, biokompatiblen und elektrisch isolierenden Verkapselung ausgeführt.

Bevorzugt werden bei dem vorstehenden nicht erfindungsgemäßen Verfahren dessen Schritte zur Vorausdetektion einer Beeinträchtigung passivierender Eigenschaften an einem noch stabil passivierten Gesamtsystem durchgeführt.

Ferner betrifft die Erfindung ein Fehleranalyse-System nach Anspruch 1, umfassend eine Erfassungsvorrichtung zum Erfassen eines Zustands einer Verkapselung und/oder einer Passivierungsschicht der Verkapselung, wobei die Verkapselung ein Mehrschichtsystem aus mehreren wechselweise übereinander liegend angeordneten Passivierungsschichten und zwischen den Passivierungsschichten liegend angeordneten, elektrisch ankontaktierten Zwischenschichten bildet, das Mehrschichtsystem dazu angeordnet ist, ein von der Verkapselung umgebenes Implantat zu schützen, und eine oberste Schicht und eine unterste Schicht des Mehrschichtsystems durch jeweils eine oberste und eine unterste Passivierungsschicht gebildet wird, wobei eine Messeinrichtung zum Durchführen einer elektrischen Messung zwischen einem Referenzpotenzial und zumindest einer elektrisch ankontaktierten Zwischenschicht und zum Erfassen zumindest eines zwischen dem Referenzpotenzial und der zumindest einen elektrisch ankontaktierten Zwischenschicht fließenden Stroms ausgebildet und vorgesehen ist und eine Vergleichseinrichtung zum Vergleichen des zumindest einen erfassten Stroms mit zumindest einem vorbestimmten Schwellenwert ausgebildet und vorgesehen ist, wobei ein Unterschreiten oder ein Überschreiten des zumindest einen Schwellenwerts einen funktionellen Zustand einer zu der zumindest einen elektrisch ankontaktierten Zwischenschicht benachbarten Passivierungsschicht anzeigt.

Die Erfindung wird nachstehend mit weiteren Vorteilen und Wirkungen anhand bevorzugter Ausführungsbeispiele unter Bezugnahme auf die Zeichnung näher beschrieben. Es zeigen:
Fig. 1 schematisch und ausschnittsweise ein mehrlagiges bzw. mehrschichtiges Passivierungssystem für beispielsweise ein Implantat mit elektrischem Zugang zu zwei elektrisch ankontaktierten Zwischenschichten in der Passivierung über einer Elektrode;
Fig. 2 schematisch ein Prinzip der Messung an passivierenden Schichten des Passivierungssystems von Fig. 1;
Fig. 3 schematisch Messsignalverläufe von elektrischen Messungen (Gleichsignalmessungen: Referenz/PBS - obere Zwischenschicht, Referenz/PBS - untere Zwischenschicht, Referenz/PBS - Implantatelektrode) an dem Implantat von Fig. 1;
Fig. 4 schematisch Messsignalverläufe von elektrischen Messungen (Gleichsignalmessungen: Referenz/PBS - obere Zwischenschicht, untere Zwischenschicht - obere Zwischenschicht, untere Zwischenschicht - Implantatelektrode) an dem Implantat von Fig. 1;
Fig. 5 schematisch Messsignalverläufe von elektrischen Messungen (Gleichsignalmessungen: obere Zwischenschicht - untere Zwischenschicht, untere Zwischenschicht - obere Zwischenschicht, sowie Referenz/PBS gegen jeweils obere Zwischenschicht, untere Zwischenschicht und Implantatelektrode) an dem Implantat von Fig. 1 zur insbesondere Produktionsausschussdetektion; und
Fig. 6 auszugweise und vereinfacht ein Ablaufdiagramm eines nicht erfindungsgemäßen Verfahrens zur Zustandserfassung an Passivierungsschichten.

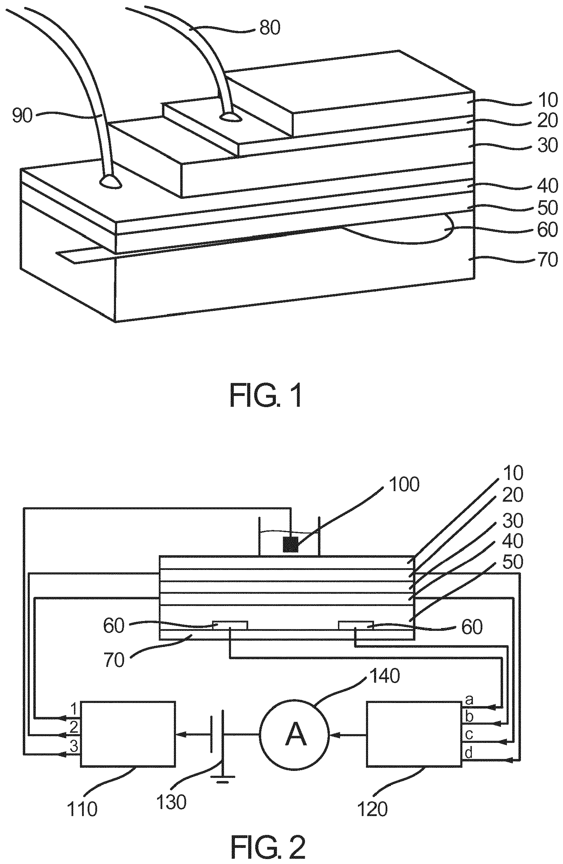

Gleiche oder funktionell äquivalente Merkmale sind in den einzelnen Figuren mit denselben Bezugszeichen versehen und werden zweckmäßig nicht redundant beschrieben. Figuren sind nicht notwendigerweise als maßstäblich zu betrachten. Eine Beschränkung auf in den Figuren angegebene Maßeinheiten besteht nicht. Fig. 1 zeigt schematisch für einen Aufbau einer einseitigen Passivierung einen elektrischen Zugang zu zwei Zwischenlagen in einer Passivierung über einer Elektrode in einem Mehrschichtsystem oder Mehrlagensystem für ein flexibles Implantat.

In Fig. 1 bezeichnet das Bezugszeichen 10 eine oberste oder erste Passivierungslage bzw. Passivierungsschicht, bezeichnet das Bezugszeichen 20 eine aus einem leitfähigen Material erzeugte erste Zwischenlage bzw. Zwischenschicht, bezeichnet das Bezugszeichen 30 eine mittlere oder zweite Passivierungslage, bezeichnet das Bezugszeichen 40 eine ebenfalls aus einem leitfähigen Material erzeugte zweite Zwischenlage, bezeichnet das Bezugszeichen 50 eine untere oder dritte Passivierungslage, und bezeichnet das Bezugszeichen 60 eine (schematisch angedeutete) Elektrode bzw. einen Erfassungspunkt eines Implantats, welche(r) durch das passivierende Mehrschichtsystem bzw. Multilagensystem 10, 30, 50 geschützt ist. In anderen Worten schützt die in Fig. 1 schematisch angedeutete Passivierung das Implantat, modellhaft angedeutet durch die Elektrode 60, vor vielfältigen negativen Degradationseffekten. Mit dem Bezugszeichen 70 ist modellhaft beispielsweise eine Art Substrat oder ein Teil des Implantats angedeutet.

Unter "modellhaft" ist hierin zu verstehen, dass ein Implantat nicht notwendigerweise tatsächlich eine "Elektrode" im eigentlichen Sinne aufzuweisen braucht, diese Begrifflichkeit jedoch zur Beschreibung von Zusammenhängen hilfreich sein kann. Während praktisch bevorzugt die elektrische Messung zwischen (beispielsweise zwei) Zwischenschichten durchgeführt wird, kann in dem hierin gezeigten beispielhaften System eine Elektrode dafür zweckentfremdet werden. In dem gemäß dem Ausführungsbeispiel verwendeten Substrat wurde beispielsweise die Elektrode für die Messung zweckentfremdet. Unter einem leitfähigem Material ist hierin beispielsweise ein Metall, ein leitfähiger Halbleiter oder allgemein ein elektrisch leitfähiges Material bzw. ein entsprechender Werkstoff zu verstehen.

Elektrische Ankontaktierungen 80, 90 an jeweils der ersten Zwischenlage 20 und der zweiten Zwischenlage 40, die auch als Bottom-Layer (unten) bzw. Top-Layer (oben) bezeichnet werden, erlauben hierbei eine Analyse jeweils einer passivierenden Schicht, beispielsweise einer Polyimid-Schicht bzw. Lage oder Layer.

Fig. 2 zeigt schematisch ein Prinzip der Messung an passivierenden Schichten des Passivierungssystems von Fig. 1. Das heißt, normalerweise wird die funktionelle Eigenschaft der Passivierung mittels einer in eine elektrisch aktive bzw. leitende Flüssigkeit über das Implantat gesetzte Referenzelektrode 100 getestet, wie in Fig. 2 dargestellt. Durch Impedanzmessungen gegenüber passivierten Bauteilen, in diesem Fall das Mehrschichtsystem bzw. Teile desselben, kann sodann eine Aussage über die eigentliche Passivierung getroffen werden. Insbesondere lassen sich über die elektrische Ankontaktierung 80, 90 der Zwischenschichten 20, 40 vielfältige Aussagen sowohl während der Produktion als auch im späteren Betrieb des Implantats treffen. Die Bezugszeichen 110 und 120 in Fig. 2 bezeichnen beispielsweise Schaltvorrichtungen (Umschalter) zur Verbindung einer Gleichsignalquelle 130 für Messspannung bzw. Messstrom und eines Strommessgeräts 140 mit jeweils anzusprechenden Komponenten bzw. Schichten oder Lagen des Mehrschichtsystems.

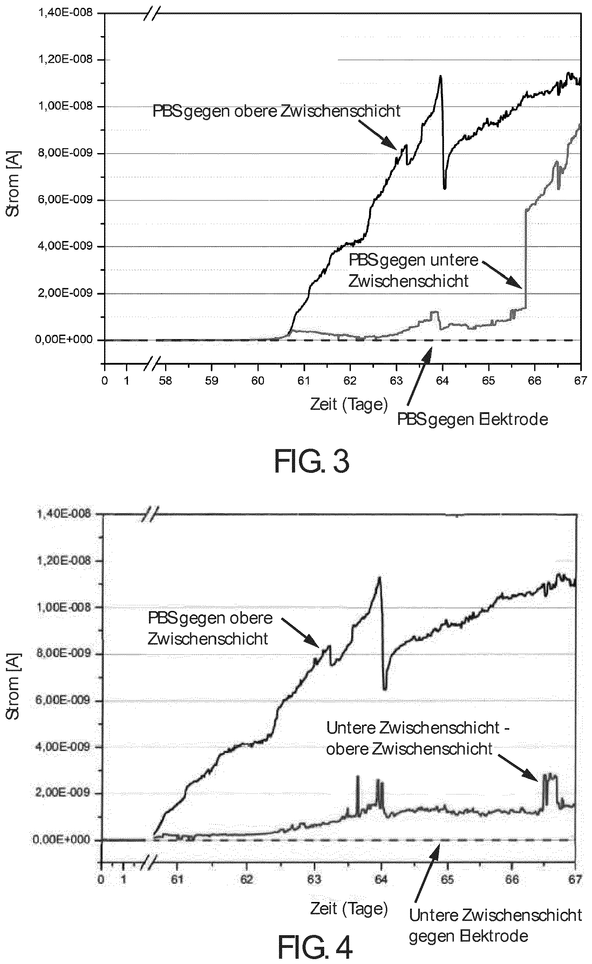

Nachstehend werden beispielhafte Messungen an einer passivierenden Verkapselung eines Implantats unter Bezugnahme auf Fig. 3 bis 5 beschrieben, wobei die in Fig. 3 bis 5 gezeigten Messungen lediglich Referenzcharakter aufweisen, da sie gegen eine sich ex vivo außerhalb eines Implantats befindende Referenzelektrode 100 durchgeführt wurden. Es wird jedoch angemerkt, dass ein sich in vivo befindendes Implantat selbst eine äquivalente Aussage über den Zustand der Passivierungslagen 10, 30, 50 auf der Grundlage der ersten und/oder zweiten Zwischenlagen 20, 40 aus einem leitfähigen Material als Referenz treffen kann.

Fig. 3 zeigt schematisch Messsignalverläufe einfacher elektrischer Messungen an dem Implantat von Fig. 1. Die elektrischen Messungen können z.B. mit einer Spannung von 1 V von der Referenzelektrode (PBS) 100 in der elektrisch aktiven Flüssigkeit gegen die beiden Zwischenlagen 20, 40 aus leitfähigem Material sowie die Elektrode 60 des Implantats durchgeführt werden. Ist kein messbarer Strom von der Referenzelektrode 100 gegen die Implantatelektrode 60 erfassbar, so kann daraus geschlossen werden, dass das gesamte passivierende System noch stabil ist. Fig. 3 zeigt auf dieser Grundlage im Einzelnen, dass in den ersten 60 Tagen nach Beginn der Messungen kein Strom zwischen der Referenz 100 und der Implantatelektrode 60 messbar und das Gesamtsystem damit stabil passiviert ist. PBS steht für beispielsweise Phosphate Buffer Solution bzw. Phosphate Buffered Saline, d.h. eine phosphatgepufferte Salzlösung. PBS und deren Anwendbarkeit sind insoweit bekannt und wird daher hierin nicht redundant beschrieben.

In der Praxis allerdings kann die oberste passivierende (Polyimid-)Schicht 10 nach etwa ca. 60 Tagen in vivo ihre passivierende Eigenschaft verlieren, wie in Fig. 3 durch die messbar ansteigenden Ströme nach etwa 60,5 Tagen dargestellt. Dieser Eigenschaftsverlust bzw. ein dadurch verursacht ansteigender Strom über eine jeweils betrachtete Messstrecke (Referenz 100 - Passivierungsschicht 10 - obere Zwischenschicht 20, Referenz 100 - mittlere Passivierungsschicht 30 - untere Zwischenschicht 40) ist sodann aufgrund der elektrischen Ankontaktierung 80 derselben über die obere, erste Zwischenlage 20 (Top-Layer) sofort messbar. Damit einhergehend ist ferner eine nennenswerte, sofortige Stromdetektion auf der unteren, zweiten Zwischenlage 40 (Bottom-Layer). In diesem Zustand ist, wie in Fig. 3 dargestellt, noch immer kein Stromfluss zwischen der Referenz 100 und der Implantatelektrode 60 erfassbar. Das heißt, das Gesamtsystem als solches ist noch immer hinreichend passiviert (über die unterste Passivierungsschicht 50), obwohl die Passivierungswirkung der Passivierungsschichten 10, 30 in der Verkapselung bereits messbar beeinträchtigt ist. Ein ansteigender Messstrom in Fig. 3 bedeutet eine mit der Zeit (und zwischen einzelnen Schichten ggf. zeitversetzt) fortschreitende Degradation einer jeweiligen Passivierungsschicht.

Fig. 4 zeigt eine Referenzmessung aus der Flüssigkeit bzw. von der Referenzelektrode 100 zu der oberen, ersten Zwischenschicht 20 sowie zwei elektrische Messungen ausschließlich innerhalb des Implantats, d.h. eine Messung von der unteren, zweiten Zwischenschicht 40 aus leitfähigem Material zu der oberen, ersten Zwischenschicht 20 aus leitfähigem Material und eine Messung von der unteren, zweiten Zwischenschicht 40 zu der Implantatelektrode 60. Diese Messungen können ebenfalls mit einem Gleichsignal und einer Spannung von beispielsweise 1 V erfolgen.

Wie in Fig. 4 dargestellt, kann das Implantat (über die Messungen innerhalb des Implantats) selbst eine Degradation bzw. Verschlechterung der mittleren passivierenden (Polyimid-)Schicht, d.h. der zweiten Passivierungsschicht 30, erkennen (untere Kurve in Fig. 4). Ferner ist aus der Messung in Fig. 4 ersichtlich, dass das passivierende Mehrschichtsystem trotz des Ausfalles zweier von drei Schichten als Ganzes noch stabil ist (durchbrochene, horizontal verlaufende Linie in Fig. 4), da kein messbarer Widerstand zwischen der unteren, zweiten Zwischenschicht 40 aus leitfähigem Material gegen die Elektrode 60 erfasst wird.

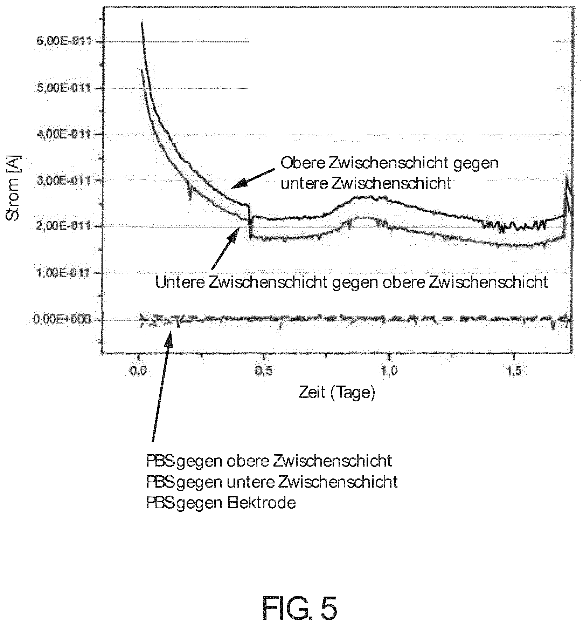

Es ist möglich, die vorgenannten Aussagen über den funktionellen Zustand der Passivierung alleine mittels des Implantats ohne Verwendung einer externen Referenz zu treffen, und damit auch nach einer Implantation des Implantats in vivo zu treffen. Dementsprechend kann eine Materialermüdung frühzeitig erkannt werden, während bzw. obwohl die passivierende Eigenschaft im Ganzen noch gegeben ist. Fig. 5 zeigt beispielhaft Gleichsignalmessungen über die erste und die zweite Zwischenschicht 20, 40. Solche Messungen sind beispielsweise zur Produktionsausschussdetektion heranziehbar. Fig. 4 zeigt anhand des Auftretens messbarer Ströme links unten in der Figur den Ausfall der zweiten passivierenden Schicht 30 mit gleichzeitig dem Ausfall der ersten passivierenden Schicht 10 nach ca. 60,5 Tagen. Die Messkurven in Fig. 5 jedoch belegen, dass ein solcher zeitgleicher Ausfall nicht überrascht und schon an einem vorbestimmten Tag 0 vorhersagbar ist. Denn obwohl das Gesamtsystem als solches als stabil passiviert messbar ist (vgl. die unteren, durchbrochenen Linien in Fig. 5 für Messungen von der Referent 100 gegen die Zwischenschichten 20, 40 und die Elektrode 60), ist gemäß Fig. 5 mittels der ersten und der zweiten Zwischenschichten 20, 40 aus leitfähigem Material bereits anfänglich ein messbarer Strom (und somit ein erfassbarer Widerstand) über die mittlere Passivierungsschicht 30 detektierbar. Die passivierende Eigenschaft dieser Schicht zeigt sich damit als von Anbeginn an nicht gesichert. Dies bestätigt sich (vgl. Fig. 4) dann auch entsprechend in einer Langzeitmessung am selben Objekt, vorzugsweise Implantat, nach in diesem Beispiel etwa 60,5 Tagen.

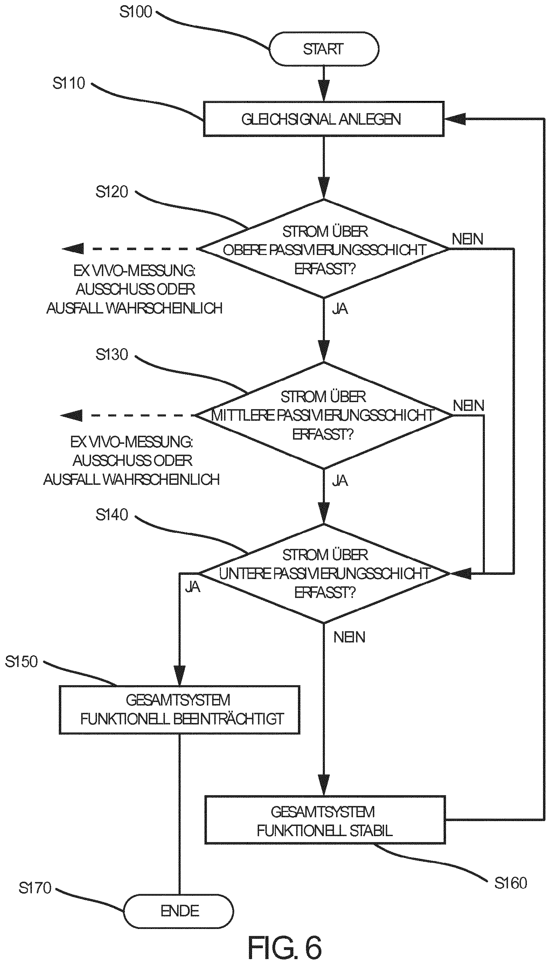

Fig. 6 zeigt ein auszugweises und vereinfachtes Ablaufdiagramm eines nicht erfindungsgemäßen Verfahrens zur Zustandserfassung an Passivierungsschichten auf Grundlage der vorstehend beschriebenen Messungen. In diesem Ausführungsbeispiel wird eine Verkapselung des Implantats mit drei Passivierungsschichten 10, 30, 50 und zwei Zwischenschichten 20, 40 angenommen. Eine Beschränkung auf eine konkrete Form und Ausgestaltung der Verkapselung besteht jedoch nicht. Beispielsweise kann die Anzahl der einzelnen Schichten höher oder geringer sein. Es versteht sich ferner, dass technische Mittel wie etwa Sensoren, Verarbeitungs- und Steuereinheiten, Speicher, Schnittstellen und dergleichen, zur Durchführung des Verfahrens an sich bekannt sind und daher eine insoweit redundante Beschreibung entfallen kann.

Nach Beginn des Verfahrens in einem Schritt S100 werden, wie vorstehend beschrieben wurde, in einem Schritt S110 an einer jeweils durchzuführenden Messung beteiligte Komponenten mit einem Gleichsignal beaufschlagt, beispielsweise über die Gleichsignalquelle 130 und die Schaltvorrichtungen 110, 120, und auf eine Erfassungseinrichtung, beispielsweise das Strommessgerät 140, aufgeschaltet.

In einem Schritt S120 wird geprüft, ob an der ersten bzw. oberen Passivierungsschicht 10 ein Strom erfassbar ist, d.h. die Passivierung durch diese Schicht beeinträchtigt ist.

Falls kein Strom erfassbar ist und die Passivierung durch diese Schicht als intakt bzw. stabil beurteilt werden kann (NEIN in Schritt S120), schreitet der Ablauf zu einem Schritt S140 fort.

Falls ein Strom erfassbar ist und die Passivierung durch diese Schicht als degradiert bzw. verschlechtert beurteilt werden muss (JA in Schritt S120), schreitet der Ablauf zu einem Schritt S130 fort.

Alternativ kann im Rahmen einer Produktionsausschussprüfung zu einer vorbestimmten und geeigneten Ausschussbehandlung ausgezweigt werden (durchbrochene Pfeillinie bei Schritt S120), sofern ein entsprechendes Kriterium (beispielsweise "Ausschuss, wenn oberste Passivierungsschicht 10 beeinträchtigt") anzuwenden ist.

In Schritt S130 wird geprüft, ob an der zweiten bzw. mittleren Passivierungsschicht 30 ein Strom erfassbar ist, d.h. die Passivierung durch diese Schicht beeinträchtigt ist.

Falls kein Strom erfassbar ist und die Passivierung durch diese Schicht als intakt bzw. stabil beurteilt werden kann (NEIN in Schritt S130), schreitet der Ablauf zu Schritt S140 fort.

Falls ein Strom erfassbar ist und die Passivierung durch diese Schicht als degradiert bzw. verschlechtert beurteilt werden muss (JA in Schritt S130), schreitet der Ablauf zu Schritt S140 fort.

Alternativ kann im Rahmen einer Produktionsausschussprüfung zu einer vorbestimmten und geeigneten Ausschussbehandlung ausgezweigt werden (durchbrochene Pfeillinie bei Schritt S130), sofern ein entsprechendes Kriterium (beispielsweise "Ausschuss, wenn oberste Passivierungsschicht 10 und mittlere Passivierungsschicht 30 beeinträchtigt") anzuwenden ist.

In Schritt S140 wird geprüft, ob an der dritten bzw. unteren Passivierungsschicht 50 ein Strom erfassbar ist, d.h. die Passivierung durch diese Schicht beeinträchtigt ist.

Falls kein Strom erfassbar ist und die Passivierung durch diese Schicht als intakt bzw. stabil beurteilt werden kann (NEIN in Schritt S140), schreitet der Ablauf zu Schritt S160 fort, in dem das Gesamtsystem als stabil passiviert beurteilt werden kann.

In Schritt S160 kann sodann ein Rücksprung des Ablaufs vorgesehen sein, über welchen die Zustandserfassung für ein als stabil passiviert beurteiltes Gesamtsystem solange fortgesetzt wird, bis eine vorbestimmte Degradation bzw. Beeinträchtigung erfasst wird, welche in einem aktuellen Verarbeitungsdurchlauf sodann zu Schritt 150 und weiter zum Ende des Ablaufs für das aktuell betrachtete Gesamtsystem in einem Schritt S170 verzweigt.

Falls ein Strom erfassbar ist und die Passivierung durch diese Schicht als degradiert bzw. verschlechtert beurteilt werden muss (JA in Schritt S140), schreitet der Ablauf zu Schritt S150 fort, in dem das Gesamtsystem als nicht stabil passiviert und damit funktionell beeinträchtigt beurteilt wird. Danach endet die Verarbeitung für das aktuell betrachtete Gesamtsystem in Schritt S170.

Es versteht sich, dass der in Fig. 6 gezeigte Ablauf nicht auf die konkrete Abfolge der dargestellten Schritte beschränkt ist, und dass in Abhängigkeit von praxisnahen Anforderungen zahlreiche Modifikationen, Kombinationen, Ergänzungen und Weglassungen möglich sind.

Beispielsweise zeigt das Ablaufdiagramm von Fig. 6 lediglich die Messung ausgehend von der Referenzelektrode. Es versteht sich jedoch, dass Messungen über die Zwischenschichten zur Erfassung von Produktionsausschuss oder der Güte der einzelnen Schichten durch Anlegen entsprechender Potenziale an die Zwischenschichten, wie vorstehend beschrieben wurde, in äquivalenter und analoger Weise zu den Messungen ausgehend von der Referenzelektrode durchführbar sind.

Beispielsweise ist es in Abhängigkeit anzuwendender Kriterien nicht notwendigerweise erforderlich, Schritt S140 immer durchlaufen zu müssen, sondern kann beispielsweise bereits Schritt S120 und/oder Schritt S140 zu Schritt S160 verzweigen, falls eine hinreichende (stabil intakte) Passivierung durch nur eine der Passivierungsschichten 10, 30 genügt, um das Gesamtsystem als stabil zu beurteilen.

Ferner kann weiter eine Ausführungsreihenfolge der im Einzelnen durchzuführenden Schritte geändert sein, beispielsweise falls einer Prüfung einer der Passivierungsschichten 10, 30, 50 eine höhere Priorität zukommt als anderen Passivierungsschichten.

In einer weiteren Modifikation kann die Reihenfolge beispielsweise mit fortschreitender Lebensdauer bzw. Nutzungsdauer des Implantats dynamisch geändert und/oder verkürzt werden, um beispielsweise bei Annäherung an einen Erwartungswert der Lebensdauer eine Messfrequenz zu erhöhen oder die Gleichsignalbelastung des Gesamtsystems zu verringern. Falls etwa die obere, erste Passivierungsschicht 10 und/oder die mittlere, zweite Passivierungsschicht 30 über einen vorbestimmten Zeitraum hinweg permanent als degradiert erfasst wird/werden, kann zum Beispiel Schritt S120 und/oder Schritt S130 ausgeblendet werden und kann für die verbleibende Lebensdauer des Gesamtsystems nur noch Schritt S140 abgeprüft werden.

Es versteht sich, dass die Erfindung nicht auf eine konkrete Verkapselungs- und/oder Passivierungsstruktur an einem Implantat beschränkt ist, sondern dass verschiedenartige Konfigurationen und Modifikationen denkbar sind.

Beispielsweise sind die vorstehend beispielhaft angegebenen Messungen in einer Anwendung eines mehrschichtigen Passivierungssystems auf flexible Substrate, beispielsweise ein flexibles elektrisch aktives Polyimidsubstrat, durchführbar, für welche Aussagen der beschriebenen Art sodann in vivo getroffen werden können. Die erforderliche elektrische Kontaktierung der Zwischenlagen 20, 40 kann beispielsweise über ViaPads im Substrat 70 selbst geschehen.

Ferner sind die vorstehend beispielhaft angegebenen Messungen in einer Anwendung auf ein flexibles (noch nicht abgelöstes) Substrat mit mehrlagiger Passivierung durchführbar. Beispielsweise können in diesem Fall durch ViaPads zumindest zwei zwischenliegende Lagen aus leitfähigem Material, d.h. Zwischenschichten 20, 40, elektrisch ankontaktiert sein und zusammen mit weiteren elektrischen Komponenten über beispielsweise BondPads ausgelesen werden.

Es versteht sich darüber hinaus, dass den beschriebenen Ausführungsbeispielen und nicht maßstabsgetreuen Zeichnungen lediglich beispielhafter Charakter zukommt und sich insoweit für den Fachmann Modifikationen ohne Weiteres ergeben können, ohne dass dadurch der beschreibungsgemäße Rahmen und der durch die beigefügten Ansprüche definierte Schutzumfang verlassen werden. Ebenso unterliegen äußere Formen, Dimensionen und dergleichen keinen besonderen Beschränkungen, solange durch sie die erfindungsgemäße Wirkung und Funktionalität bereitgestellt und erzielt wird. Der Schutzumfang der Erfindung durch die beigefügten Ansprüche definiert.

## Patentansprüche

1. Fehleranalyse-System mit einer Verkapselung, die dafür vorgesehen ist ein in vivo platziertes Implantat (60, 70) zu schützen, und einer Erfassungsvorrichtung welche dazu ausgebildet und vorgesehen ist, einen Zustand der Verkapselung und/oder einer Passivierungsschicht (10, 30, 50) der Verkapselung zu erfassen, wobei die Verkapselung ein Mehrschichtsystem aus mehreren wechselweise übereinander liegend angeordneten Passivierungsschichten (10, 30, 50) und zwischen den Passivierungsschichten (10, 30, 50) liegend angeordneten, elektrisch ankontaktierten Zwischenschichten (20, 40) bildet, das Mehrschichtsystem dafür ausgebildet ist, das von der Verkapselung umgebenes Implantat (60, 70) zu schützen, und eine oberste Schicht (10) und eine unterste Schicht (50) des Mehrschichtsystems durch jeweils eine oberste (10) und eine unterste (50) Passivierungsschicht gebildet wird, wobei die Erfassungsvorrichtung umfasst:
- eine Messeinrichtung, ausgebildet und vorgesehen zum Durchführen einer elektrischen Messung zwischen einem Referenzpotenzial und der zumindest einen elektrisch ankontaktierten Zwischenschicht (20, 40) der Verkapselung und zum Erfassen zumindest eines zwischen dem Referenzpotenzial und der zumindest einen elektrisch ankontaktierten Zwischenschicht (20, 40) fließenden Stroms; und
- einer Vergleichseinrichtung, ausgebildet und vorgesehen zum Vergleichen des zumindest einen erfassten Stroms mit zumindest einem vorbestimmten Schwellenwert, wobei ein Unterschreiten oder ein Überschreiten des zumindest einen Schwellenwerts einen funktionellen Zustand einer zu der zumindest einen elektrisch ankontaktierten Zwischenschicht (20, 40) benachbarten Passivierungsschicht (10, 30, 50) anzeigt.

2. System nach Anspruch 1, wobei
- mehrere jeweils elektrisch ankontaktierte Zwischenschichten (20, 40), die zwischen jeweils zwei Passivierungsschichten (10, 30, 50) der Verkapselung angeordnet sind, gegen ein gemeinsames Referenzpotenzial gemessen sind;
- für jede der mehreren Zwischenschichten (20, 40) jeweils ein Messstrom erfasst wird, der einer entsprechenden der Passivierungsschichten (10, 30, 50) zugeordnet ist und deren funktionellen Zustand anzeigt; und
- jeder Messstrom mit jeweils einem vorbestimmten Schwellenwert verglichen wird, wobei ein Überschreiten des vorbestimmten Schwellenwerts einen beeinträchtigten Zustand der jeweils zugeordneten Passivierungsschicht (10, 30, 50) anzeigt.

3. System nach Anspruch 1 oder 2, wobei
- das Referenzpotential für die elektrische Messung eine Elektrode (60) des zu schützenden Implantats (60, 70) ist und
- der vorbestimmte Schwellenwert null ist und ein Vergleich erfasster Messströme über mehrere Schichtfolgen hinweg einen Gesamtzustand des die Verkapselung bildenden Mehrschichtsystems anzeigt, wobei dann, wenn der Vergleich anzeigt, dass zumindest zwischen der Elektrode (60) des zu schützenden Implantats (60, 70) und einer von dieser durch die unterste Passivierungsschicht (50) getrennte Zwischenschicht (40) kein Messstrom erfassbar ist, unabhängig von einem erfassten Zustand höher liegender Passivierungsschichten (10, 30), auf funktionelle Stabilität des Gesamtsystems geschlossen wird.

4. System nach einem der vorangehenden Ansprüche, wobei
- der vorbestimmte Schwellenwert null ist und ein Vergleich erfasster Messströme über mehrere Schichtfolgen hinweg einen Gesamtzustand des die Verkapselung bildenden Mehrschichtsystems anzeigt, wobei dann, wenn das Mehrschichtsystem zumindest eine obere (10), eine mittlere (30) und eine untere (50) Passivierungsschicht beinhaltet, und wenn in einer anfänglichen Systemprüfmessung nach Produktionsende des Gesamtsystems ein messbarer Strom zwischen einer ersten Zwischenschicht (20), die zwischen der oberen (10) und der mittleren (30) Passivierungsschicht angeordnet ist, und einer zweiten Zwischenschicht (40), die zwischen der mittleren (30) und der unteren (50) Passivierungsschicht angeordnet ist, über die mittlere Passivierungsschicht (30) erfasst wird, auf eine Ausschuss bedingende Grundbeeinträchtigung zumindest der mittleren Passivierungsschicht (30) und/oder des Gesamtsystem geschlossen wird.

5. System nach einem der vorangehenden Ansprüche, wobei
- die Verkapselung dazu angeordnet ist, das Implantat (60, 70), vorzugsweise ein flexibles Implantat, in vivo fluiddicht und/oder gasdicht zu kapseln, und das System dazu konfiguriert ist, anhand einer Analyse und Bewertung zumindest einer passivierenden Schicht durch elektrische Messung eine in vivo Fehleranalyse und/oder Gesundheitsanalyse der Implantatpassivierung und/oder Ermittlung von Produktionsausschuss unter Entfall externer Referenzkomponenten durchzuführen.

6. System nach einem der vorangehenden Ansprüche, wobei
- die elektrische Messung externreferenzfrei zwischen zwei Zwischenschichten (20, 40) durchgeführt ist.

7. System nach einem der vorangehenden Ansprüche, wobei
- die Verkapselung biostabil, biokompatibel und elektrisch isolierend ist.

8. System nach einem der vorangehenden Ansprüche, wobei
die Messung und das Vergleichen zur frühzeitigen Erfassung einer Verschlechterung passivierender Eigenschaften an einem noch stabil passivierten Gesamtsystem durchgeführt sind.

## Claims

1. A failure analysis system comprising an encapsulation provided to protect an implant (60, 70) placed in vivo and a detection device configured and provided to detect a status of the encapsulation and/or of a passivation layer (10, 30, 50) of the encapsulation, wherein the encapsulation forms a multi-layer system from multiple passivation layers (10, 30, 50) arranged to lie alternatingly on top of one another and electrically contacted intermediate layers (20, 40) arranged to lie between the passivation layers (10, 30, 50), the multi-layer system is configured to protect an implant (60, 70) surrounded by the encapsulation, and a top layer (10) and a bottom layer (50) of the multi-layer system are formed by a respective top (10) and a bottom (50) passivation layer, wherein the detection device comprises the following:
- a measuring device that is configured and provided for carrying out an electrical measurement between a reference potential and the at least one electrically contacted intermediate layer (20, 40) of the encapsulation, and for detecting at least one current flowing between the reference potential and the at least one electrically contacted intermediate layer (20, 40); and
- a comparison device that is configured and provided for comparing the at least one detected current with at least one pre-determined threshold value, wherein, if the at least one detected current falls below or exceeds the at least one threshold value, this indicates a functional state of a passivation layer (10, 30, 50) adjacent to the at least one electrically contacted intermediate layer (20, 40).

2. The system according to claim 1, wherein
- a plurality of electrically contacted intermediate layers (20, 40) interposed between two respective passivation layers (10, 30, 50) of the encapsulation are measured against a common reference potential;
- for each of the multiple intermediate layers (20, 40), a respective measuring current is detected which is assigned to a corresponding one of the passivation layers (10, 30, 50) and indicates the functional state thereof; and
- each measuring current is compared to a respective pre-determined threshold value, wherein exceeding of the pre-determined threshold value indicates an impaired status of the respective assigned passivation layer (10, 30, 50).

3. The system according to claim 1 or 2, wherein
- the reference potential for the electrical measurement is an electrode (60) of the implant (60, 70) to be protected, and
- the pre-determined threshold value is zero and a comparison of detected measuring currents over a plurality of layer sequences indicates an overall status of the multi-layer system forming the encapsulation, wherein, if the comparison indicates that at least between the electrode (60) of the implant (60, 70) to be protected and an intermediate layer (40) separated from the electrode (60) by the bottom passivation layer (50) no measuring current can be detected, irrespective of a determined status of higher passivation layers (10, 30) a functional stability of the overall system is concluded.

4. The system according to one of the preceding claims, wherein
- the pre-determined threshold value is zero and a comparison of detected measuring currents over a plurality of layer sequences indicates an overall status of the multi-layer system forming the encapsulation, wherein if the multi-layer system includes at least one top (10), one central (30) and one bottom (50) passivation layer and if in an initial system inspection measurement after the end of production of the overall system a measurable current is detected via the central passivation layer (30) between a first intermediate layer (20) interposed between the top (10) and the central (30) passivation layers and a second intermediate layer (40) interposed between the central (30) and the lower (50) passivation layers, a basic impairment of at least the central passivation layer (30) and/or of the overall system which causes reject will be concluded.

5. The system according to one of the preceding claims, wherein
- the encapsulation is arranged to encapsulate the implant (60, 70), preferably a flexible implant, in vivo in a fluid-tight and/or gas-tight manner, and the system is configured to carry out, by way of analysis and evaluation of at least one passivating layer by electrical measurement, an in vivo failure analysis and/or health analysis of the implant passivation and/or detection of production reject while external reference components are omitted.

6. The system according to one of the preceding claims, wherein
- the electrical measurement is carried out free from external references between two intermediate layers (20, 40).

7. The system according to one of the preceding claims, wherein
- the encapsulation is biostable, biocompatible and electrically insulating.

8. The system according to one of the preceding claims, wherein the measurement and the comparison for timely detection of degradation of passivating properties on a still stably passivated overall system.

## Revendications

1. Système d'analyse d'erreur avec une encapsulation, qui est destinée à protéger un implant (60, 70) placé in vivo, et un dispositif de détection, lequel est réalisé pour et destiné à détecter un état de l'encapsulation et/ou d'une couche de passivation (10, 30, 50) de l'encapsulation, dans lequel l'encapsulation forme un système multicouche composé de plusieurs couches de passivation (10, 30, 50) disposées de manière à se situer en alternance les unes sur les autres et couches intermédiaires (20, 40) disposées de manière à se situer entre les couches de passivation (10, 30, 50), mises en contact électrique, le système multicouche est réalisé pour protéger l'implant (60, 70) entouré par l'encapsulation, et une couche la plus haute (10) et une couche la plus basse (50) du système multicouche est formée par respectivement une couche de passivation la plus haute (10) et une la plus basse (50), dans lequel le dispositif de détection comprend :
- un appareil de mesure, réalisé pour et destiné à mettre en œuvre une mesure électrique entre un potentiel de référence et la au moins une couche intermédiaire (20, 40) mise en contact électrique de l'encapsulation et à détecter au moins un courant circulant entre le potentiel de référence et la au moins une couche intermédiaire (20, 40) mise en contact électrique ; et
- un appareil de comparaison, réalisé pour et destiné à comparer le au moins un courant détecté à au moins une valeur seuil prédéfinie, dans lequel un passage au-dessous ou un dépassement de la au moins une valeur seuil indique un état fonctionnel d'une couche de passivation (10, 30, 50) voisine de la au moins une couche intermédiaire (20, 40) mise en contact électrique.

2. Système selon la revendication 1, dans lequel
- plusieurs couches intermédiaires (20, 40) respectivement mises en contact électrique, qui sont disposées entre respectivement deux couches de passivation (10, 30, 50) de l'encapsulation, sont mesurées par rapport à un potentiel de référence commun ;
- pour chacune des plusieurs couches intermédiaires (20, 40) respectivement un courant de mesure est détecté, qui est associé à une correspondante des couches de passivation (10, 30, 50) et indique l'état fonctionnel de celles-ci ; et
- chaque courant de mesure est comparé à respectivement une valeur seuil prédéfinie, dans lequel un dépassement de la valeur seuil prédéfinie indique un état altéré de la couche de passivation (10, 30, 50) respectivement associée.

3. Système selon la revendication 1 ou 2, dans lequel
- le potentiel de référence pour la mesure électrique est une électrode (60) de l'implant (60, 70) à protéger et
- la valeur seuil prédéfinie est nulle et une comparaison de courants de mesure détectés par-dessus plusieurs suites de couches indique un état global du système multicouche formant l'encapsulation, dans lequel, lorsque la comparaison indique qu'aucun courant de mesure ne peut être détecté au moins entre l'électrode (60) de l'implant (60, 70) à protéger et une couche intermédiaire (40) séparée de celle-ci par la couche de passivation (50) la plus basse, indépendamment d'un état détecté de couches de passivation (10, 30) situées plus haut, on conclut alors à la stabilité fonctionnelle du système global.

4. Système selon l'une quelconque des revendications précédentes, dans lequel
- la valeur seuil prédéfinie est nulle et une comparaison de courants de mesure détectés par-dessus plusieurs suites de couches indique un état global du système multicouche formant l'encapsulation, dans lequel lorsque le système multicouche comporte au moins une couche de passivation supérieure (10), une centrale (30) et une inférieure (50), et lorsqu'au début d'une mesure de vérification de système après la fin de la production du système global un courant mesurable est détecté entre une première couche intermédiaire (20), qui est disposée entre la couche de passivation supérieure (10) et la centrale (30), et une deuxième couche intermédiaire (40), qui est disposée entre la couche de passivation centrale (30) et l'inférieure (50), par le biais de la couche de passivation centrale (30), on conclut alors à une dégradation fondamentale au moins de la couche de passivation centrale (30) et/ou du système global entraînant un rej et.

5. Système selon l'une quelconque des revendications précédentes, dans lequel
- l'encapsulation est disposée pour encapsuler in vivo l'implant (60, 70), de préférence un implant flexible, de manière étanche au fluide et/ou étanche au gaz, et le système est configuré pour réaliser sur la base d'une analyse et évaluation au moins d'une couche de passivation par mesure électrique une analyse d'erreur et/ou analyse de santé in vivo de la passivation d'implant et/ou détermination d'un rejet de production en l'absence de composants de référence externes.

6. Système selon l'une quelconque des revendications précédentes, dans lequel
- la mesure électrique est réalisée de manière exempte de référence externe entre deux couches intermédiaires (20, 40).

7. Système selon l'une quelconque des revendications précédentes, dans lequel
- l'encapsulation est biostable, biocompatible et électriquement isolante.

8. Système selon l'une quelconque des revendications précédentes, dans lequel
la mesure et la comparaison pour la détection précoce d'une dégradation de propriétés de passivation sont réalisées sur un système global encore passivé de manière stable.
